(19) Europäisches Patentamt / European Patent Office / Office européen des brevets

(11) **EP 4 485 372 A1**

(12) **EUROPEAN PATENT APPLICATION**

(43) Date of publication:
**01.01.2025 Bulletin 2025/01**

(21) Application number: **23182186.9**

(22) Date of filing: **28.06.2023**

(51) International Patent Classification (IPC):
**G06V 10/62** (2022.01)      **G06V 40/10** (2022.01)
**G06V 40/20** (2022.01)

(52) Cooperative Patent Classification (CPC):
**G06V 40/20; G06V 10/62; G06V 40/103; G06V 40/23**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **TOYOTA JIDOSHA KABUSHIKI KAISHA**
**Toyota-shi, Aichi-ken, 471 8571 (JP)**

(72) Inventors:
• **OPREA, Georgeta-Madalina**
**1140 Brussels (BE)**

• **ABDELKAWY, Hazem**
**1140 Brussels (BE)**
• **CULLIGAN, Jay**
**1140 Brussels (BE)**

(74) Representative: **Cabinet Beau de Loménie**
**158, rue de l'Université**
**75340 Paris Cedex 07 (FR)**

Remarks:
Amended claims in accordance with Rule 137(2) EPC.

(54) **COMPUTER-IMPLEMENTED METHOD FOR POSTURE DETERMINATION AND COMPUTING SYSTEM**

(57) A computer-implemented method for posture determination is presented. The method includes: a training stage of generating at least one distribution from first pieces of information representing a class of poses of one or more training subjects; and an inference stage including steps of: obtaining at least one second piece of information representing at least one pose of an inference subject; and determining whether the inference subject has a good or bad posture using a statistical anomaly detection technique based on the at least one second piece of information and the at least one distribution .

FIG.3

**EP 4 485 372 A1**

S504B

Timer 409 has expired

S502A

Yes

Anomaly ≥ Ta      Bad posture timer      S504A

S502B                    S503A

S501      No      409 hasn't started

S502C      Movement < Tm

Anomaly < Ta      Stillness timer 408      S504C

S503B

hasn't started

Timer 408 has expired

S504D

Movement ≥ Tm      S503C

# FIG.5

**Description**

BACKGROUND OF THE INVENTION

Field of the Invention

The invention relates to the field of posture detection, and in particular, to a method for posture determination and a computing system.

Description of Related Art

Human posture detection techniques aim to understand and analyze the posture or position of the human body. They serve as valuable tools across various fields, including video surveillance, human-computer interaction, sports analysis, virtual reality, entertainment (e.g., video games and animation), as well as health-related applications such as physiotherapy, ergonomics, and injury prevention. Existing methods for posture monitoring, designed to assist users in enhancing their seated posture, principally fall into two categories: sensor-based methods and pose-estimation based methods.

Sensor-based methods incorporate one or more sensors affixed directly to the body or to the surface where a person is sitting. These sensors, which can include accelerometers, gyroscopes, and pressure sensors, gather data about body position and movement. This raw data is subsequently processed and leveraged to train a model that can classify postures as good, poor, or in some cases, the severity of poor posture. On the other hand, pose estimation-based methods leverage computer vision techniques. Typically employing generalist models that use supervised learning strategies, these methods analyze images or videos to determine the three-dimensional coordinates of key body points. These points are typically associated with bodily joints. The derived coordinates facilitate the computation of the angles between joints, which can then be analyzed and used to evaluate postures as good or poor.

However, these methods can be expensive to implement, computationally demanding, or time-consuming.

SUMMARY OF THE INVENTION

To solve the problem described above, the invention presents a novel posture determination method that utilizes statistical anomaly detection, and a computing system configured to implement the method.

As will be appreciated by one skilled in the art, aspects of the present disclosure can be embodied as a system, method, computer program or computer readable medium. Accordingly, aspects of the present disclosure can take the form of an entirely hardware embodiment, an entirely software embodiment (including firmware, resident software, micro-code, and so forth), or an embodiment combining software and hardware aspects. In this context, the term "module" herein can refer to a software component, a hardware component, or a combination of software and hardware components. Besides, various embodiments, examples and optional implementations described herein are compatible with each other unless otherwise stated.

In a first aspect, the disclosure is directed to the method for posture determination. The method includes a training stage of generating at least one distribution from first pieces of information representing a class of poses of one or more training subjects, and an inference stage including a both step of obtaining at least one second piece of information representing at least one pose of an inference subject, and a step of determining whether the inference subject has a good or bad posture using a statistical anomaly detection technique based on the at least one second piece of information and the at least one distribution.

As known in the art, a statistical anomaly detection technique may be an approach used in data analysis that identifies data points deviating significantly from expected pattern(s) inherent within one or more distributions in a dataset. For example, a statistical anomaly detection technique may identify a data point that falls many standard deviations away from the mean as an anomaly.

As used herein, the labels "good" or "bad" denote postures that respectively meet or fail to meet certain specific standards, such as those set by ergonomics. A "good" posture aligns with these standards, being deemed satisfactory or beneficial, while a "bad" posture is considered undesired or detrimental.

As such, the method for posture determination, devoid of the need for direct sensor attachment to the body or the surface where a person sits, circumvents the requirement for labeled data. As a result, it is able to determine whether the inference subject has a good or bad posture in a manner that is time-efficient, computationally undemanding, and cost-effective.

The term "subject" denotes an entity to which the first or second pieces of information pertain, potentially a human individual. To further bolster the accuracy of the method, the one or more training subjects may include the inference subject. In a particular example, the first pieces of information represent the class of poses of one training subject, which is the same as the inference subject, so as to enable a user-specific and personalized posture determination method that performs well for specific individuals with unique posture habits or bodily features.

In another example where the one or more training subjects includes the inference subject, the inference stage further includes a step of receiving identity information of the inference subject, and a step of retrieving the distribution of information representing the class of poses of the inference subject using the identity information.

In still another example, the class of poses represents one or more good/ideal postures adopted by the one or more training subjects. Further, the class of poses may represent a good posture adopted by one training subject, which is the same as the inference subject. For instance, the class of poses could encompass poses of the training subject that represent a correct sitting posture at a workstation or an optimal posture while occupying a driver's seat.

In still another example, the quantity of the at least one second piece of information equals the size of a dimension of an anomaly window. This size may be designated as Z. The anomaly window may be one-dimensional or multiple-dimensional. In particular, each anomaly window following the initial one may start immediately after the preceding anomaly window ends. Or, the anomaly window may be a sliding window, which shifts L second piece(s) of information at a time, where L is a positive integer and is smaller than Z. The anomaly window could move through second pieces of information that represent poses of the inference subject. L may be one so as to cover every possible grouping of Z successive second piece(s) of information.

Accordingly, the determining step may be performed for each of multiple anomaly windows based on the at least one distribution and the Z second piece(s) of information within the anomaly window. This could reduce the computational costs, prevent unnecessary and frequent disturbance to the user, ensure timely detection of poor posture or lack of movement, and/or, guarantee the timely delivery of reminders to the user.

In still another example, each of the first pieces of information includes an image obtained by using a camera, herein referred to as a training image. In addition to or as an alternative to this example, each of the at least one second piece of information includes an image obtained by using a camera, herein referred to as an inference image. Each of the training images and/or the at least one inference image can be a frame of a video recorded by the camera, or can be an individual photo captured by the camera. In particular, each image may be a video frame livestreamed into the system,

In addition, the camera configured to capture the training images and/or the inference image(s) may be a depth camera, or a two-dimensional ("2D) camera that captures images or video without depth information. As known in the art, a depth camera can measure the distance from the camera to the objects in its field of view. This information can be used to create a depth map, which is a representation of the distances to all the objects in the scene. For instance, the camera is an RGBD camera, which is a type of depth camera that captures RGB images along with per-pixel depth information.

In still another example, the determining step further includes a step of converting the at least one second piece of information to at least one anomaly score based on the at least one distribution, and a step of determining whether the inference subject has the good posture or the bad posture based on the at least one anomaly score.

In particular, the converting step may include a step of deriving at least one feature from the at least one second piece of information and a step of converting the at least one feature to the at least one anomaly score based on the at least one distribution. Each of the at least one feature is associated with a key body part of the inference subject The key body part is also called a "joint". In addition, at least one feature each associated with a joint of one of the one or more training subjects may be derived from each of the first pieces of information.

Further, the deriving step may include a step of analyzing the at least one second piece of information using a pose estimation technique to produce at least one result, and a step of deriving the at least one feature from the at least one result.

Additionally or alternatively, the at least one feature may include at least one angle. In particular, the at least one angle may be derived from at least one quaternion that is included in or derived from the at least one second piece of information. For example, the at least one quaternion may be produced by applying the pose estimation technique to analyze the at least one second piece of information. And/or, each of the at least one angle may represent an angle between two body segments connected by the joint associated with the angle. For example, an angle associated with an elbow joint represents the angle formed between the corresponding shoulder and the elbow. The at least one feature may include any other type of feature(s) that can represent the pose of the interface subject.

Additionally or alternatively, the converting step may include: converting one of the at least one feature that is associated with an $i^{th}$ joint of the inference subject into a joint-specific anomaly score, based on a relationship between the standard deviation of a distribution included in the at least one distribution and a difference between the feature and a mean of the distribution. The distribution includes features associated with the $i^{th}$ joint of the one or more training subjects. In particular, these features included in the distribution and the feature which is converted to the score may be the same kind of features, such as angels that are derived from quaternions produced by a pose estimation model.

Additionally or alternatively, the step of converting the at least one feature to the at least one anomaly score may include: converting the features to an average anomaly score equaling an average of base values that is incorporated with weights. Each of the base values is determined by one of the features and one of the distributions, and the distribution and the feature are associated with a same joint. In particular, the distribution may include features that are associated with the same joint of the one or more training subjects.

For instance, the number of the obtained at least one second piece of information is N, and K features are derived from each of the N second pieces of information, where N and K are integers greater than 1. Converting the features to the average anomaly score includes: converting an $i^{th}$ feature that is associated with an $i^{th}$ joint of K joints of the inference subject and is derived from one of the N second pieces of information to a joint-specific anomaly score corresponding to the $i^{th}$ joint; calculating K mean scores each equaling an average of N joint-specific anomaly scores corresponding to one of the K joints of the inference subject; and calculating the average anomaly score, where each of the base values is one of the K mean scores.

In particular, the $K \times N$ joint-specific anomaly scores may be inserted into the anomaly window, which may be a two-dimensional array of size $K \times N$ or $N \times K$, where each element of the array represents one of the anomaly scores. The method may perform the above-described calculation steps using this array, which could make the process more efficient.

Further, the weight of each of the base values may depend on the degree that the corresponding feature weighs on the bad posture.

Alternatively or additionally, the step of determining whether the inference subject has the good posture or the bad posture based on the at least one anomaly score may be implemented based on at least one relationship between the at least one anomaly score and the at least one anomaly threshold.

In particular, the at least one anomaly score is an average anomaly score, the at least one anomaly threshold is an average anomaly threshold, and the average anomaly threshold is greater than a first maximum anomaly score derived from the at least one distribution. Or, the at least one anomaly score includes joint-specific anomaly scores each corresponding to a different joint of the inference subject, the at least one anomaly threshold includes joint-specific anomaly thresholds each corresponding to a different joint of the inference subject, and each of the joint-specific anomaly thresholds is greater than a second maximum anomaly score derived from one of the at least one distribution, where the joint-specific anomaly threshold and the distribution are associated with a same joint.

For instance, the first maximum anomaly score is the maximum standard score required to encompass majority of the data points across the at least one distribution. The standard score is the number of standard deviations by which a data point is above or below the mean of a distribution. For example, if up to three standard deviations are required to capture 99.7% of the data points across the distributions, the first maximum anomaly score is 3. Similarly, a second maximum anomaly score may be the maximum standard score required to encompass majority of the data points in the corresponding distribution.

Further, a comparison may be made between the at least one anomaly score and the at least one anomaly threshold, and the determination may be made based on one or more comparison results.

For example, the method may determine that the inference subject has the bad posture when determining that one of the joint-specific anomaly scores is greater than or equal to the corresponding joint-specific anomaly threshold, and/or, may determine that the inference subject has the good posture when determining that all the joint-specific anomaly scores are smaller than the respective joint-specific anomaly thresholds. Or, the method may determine that the inference subject has the bad posture if the average anomaly score is equal to or greater than the average anomaly threshold.

The various examples and optional implementations presented above could further simplify various steps or stages of the method, further improve the accuracy of posture determination, and/or, further reduce the cost.

In still another example, the inference stage includes a step of determining a degree of movement of the inference subject based on P second pieces of information that represent P poses of the inference subject, where P represents an integer greater than 1. The inference stage further includes a step of comparing the degree of movement with a movement threshold. This example could determine whether the inference subject has been maintaining the same posture in the past P second pieces of information and aid the user in enhancing his/her postural wellbeing. In particular, the movement threshold may be determined based on the level of physical activity required to promote optimal heart health. Or, the movement threshold may be greater than the maximum degree of movement calculated from the first pieces of information.

In the following, the disclosure provides various optional implementations of the example above that could further assist users to improve their postures.

The step of determining the degree of movement may include: determining a degree of difference across the P poses. For instance, the P second pieces of information are associated with P different time points, and the step of determining the degree of movement includes: determining a degree of difference between a pose represented by one of the P second pieces of information that is associated with a final time point of the different time points, and an average of the poses represented by the P second pieces of information.

Further, the step of determining the degree of movement may include: dividing the degree of difference between the pose represented by the second piece of information associated with the final time point and the average of the poses by P.

Still further, P may equal the size of a dimension of a movement window. The movement window may be one-dimensional or multiple-dimensional. In particular, each movement window following the initial one may start immediately after the preceding movement ends. Or, the movement window may be a sliding window, which shifts Q second piece(s) of information at a time, where Q is a positive integer smaller than P. Accordingly, the step of determining the degree of movement may be performed for each of multiple movement windows based on the P second pieces of information within the anomaly window.

Still further, the degree of difference may be represented by an average of variances that is incorporated with weights. Each of the variances measures a degree of difference between a feature and an average feature. The former feature is associated with one of K different joints of the inference subject, and is derived from the second piece of information associated with the final time point. In particular, the former may be one of those used in the above-described process of determining whether the inference subject has the good or bad posture. The average feature is associated with the same joint of the inference subject, and is derived from the P second pieces of information. K represents an integer greater than 1.

Still further, the $K \times P$ features may be inserted into the movement window, which may be an array of size $K \times P$ or $P \times K$. The method may perform the determination of the degree of difference using this array, which could make the process more efficient.

Still further, the P second pieces of information may be the obtained at least one second piece of information.

Still further, each of the variances may be a square of a difference between a feature and the average feature.

Still further, the inference stage may include a step of rendering an alert when determining that the degree of movement is smaller than a movement threshold and a timer has expired, or, a step of starting the timer when determining that the degree of movement is smaller than the movement threshold and the timer has not been started, or, a step of resetting the timer when determining that the degree of movement is greater than or equal to the movement threshold. As can be appreciated, "reset" refers to returning the timer's count back to its original value, typically zero. This does not necessarily imply that the timer is restarted. By issuing alerts when the user has maintained the same posture for an extended period, this example fosters conscious awareness and promotes healthier habits.

In particular, the timer may be a bad posture timer if a result of the step of determining whether the inference subject has the good posture or the bad posture indicates that the inference subject has the bad posture. Or, the timer may be a stillness timer if the result indicates that the inference subject has the good posture. In addition, the at least one second piece of information may be associated with at least one time point, and one of the at least one second piece of information that is associated with a last one of the at least time point may be the second piece of information that is associated with the last one of the P different time points. That is, the anomaly window and the movement window overlap and conclude with the same second piece of information.

Moreover, the alert which is rendered when the bad posture timer has expired may include information identifying the particular part(s) of the inference subject that contributes to the bad posture. The method may determine the particular part(s) according to the relationships between the joint-specific anomaly scores and the joint-specific anomaly thresholds.

While a user may engage in various sitting postures, prolonged periods of sitting are widely acknowledged as detrimental to health. To address such concerns, in still another example, the inference stage further includes a step of rendering an alert when determining that an activity timer has expired. Further, the alert may be rendered under the condition of determining that the inference subject has been sitting during an elapsed time of the activity timer based at least partially on the at least one second piece of information. This may further help promoting a healthy lifestyle.

In a second aspect, the disclosure provides a computing system configured to implement the method. The computing system includes a training model configured to generate at least one distribution from first pieces of information representing a class of poses of one or more training subjects, and an inference module including an obtaining module and a first determining module. The obtaining module is configured to obtaining at least one second piece of information representing at least one pose of an inference subject. The first determining module is configured to determine whether the inference subject has a good or bad posture using a statistical anomaly detection technique based on the at least one second piece of information and the at least one distribution.

In still another example, each of the first pieces of information includes a training image, and the training module is further configured to obtain the training images by using a camera. And/or, each of the at least one second piece of information includes an inference image, and the obtaining module is further configured to obtain the at least one inference image by using a camera. The system may include one or more cameras configured to take the training images of the one or more training subjects, and/or, to take the at least one inference image of the inference subject.

In still another example, the first determining module further includes a first converting module and a second determining module. The first converting module is configured to convert the at least one second piece of information to at least one anomaly score based on the at least one distribution. The second determining module is configured to determine whether the inference subject has the good posture or the bad posture based on the at least one anomaly score.

In particular, the first converting module may include a first deriving module configured to derive at least one feature each associated with a joint of the inference subject from the at least one second piece of information, and a second converting module configured to convert the at least one feature to the at least one anomaly scored based on the at least one distribution. In addition, the computing system may further include a second deriving module configured to derive at least one feature each associated with a joint of the one of the one or more training subjects from each of the first pieces of information.

Further, the first deriving module may include an analyzing module configured to analyze the at least one second piece of information using a pose estimation technique to produce at least one result, and a third deriving module configured to derive the at least one feature from the at least one result. The pose-estimation model may be part of the system, and may be configured to utilize 2D pose estimation technique or 3D pose estimation technique to process the at least one second piece of information. For example, the model may be the native pose estimation model of a Microsoft Kinect® motion sensing device, and the outputs of the model after processing the at least one second piece of information are normalized quaternions that represent the orientations of joints.

Additionally or alternatively, the first converting module may include a third converting module configured to convert one of the at least one feature that is associated with an $i^{th}$ joint of the inference subject into a joint-specific anomaly score, based on a relationship between the standard deviation of a distribution included in the at least one distribution and a difference between the feature and a mean of the distribution. The distribution includes features associated with the $i^{th}$ joint of the one or more training subjects.

Additionally or alternatively, the first converting module may include a fourth converting module configured to convert the features to an average anomaly score equaling an average of base values that is incorporated with weights, and a third determining module configured to determine each of the base values according to one of the features and one of the distributions that are associated with a same joint.

For instance, the number of the obtained at least one second piece of information is N, and the first deriving module is configured to derive K features from each of the N second pieces of information, where N and K are integers greater than 1. The fourth converting module further includes a fifth converting module configured to convert an $i^{th}$ feature that is associated with an $i^{th}$ joint of K joints of the inference subject and is derived from one of the N second pieces of information to a joint-specific anomaly score corresponding to the $i^{th}$ joint, a first calculating module configured to calculate K mean scores each equaling an average of N joint-specific anomaly scores corresponding to one of the K joints of the inference subject, and a second calculating module configured to calculate the average anomaly score, where each of the base values is one of the K mean scores.

Additionally or alternatively, the second determining module may be configured to determine whether the inference subject has the good posture or the bad posture based on at least one relationship between the at least one anomaly score and the at least one anomaly threshold.

In particular, the second determining module may further include a first comparison module configured to make a comparison between the at least one anomaly score and the at least one anomaly threshold, and a fourth determining module configured to determine whether the inference subject has the good posture or the bad posture based on one or more comparison results.

In still another example, the inference module further includes a fifth determining module configured to determine a degree of movement of the inference subject based on P second pieces of information that represent P poses of the inference subject, where P is an integer greater than 1. The inference module may further include a second comparison module configured to determine a relationship between the degree of movement and a movement threshold.

Further, the P second pieces of information may be associated with P different time points, and the fifth determining module may be particularly configured to determine a degree of difference between a pose represented by one of the P second pieces of information that is associated with a final time point of the P different time points, and an average of the P poses represented by the P second pieces of information.

Still further, the inference module may include a sixth determining module configured to determine whether a timer has expired or whether the timer has started. Accordingly, the inference module may further include a first rendering module configured to render an alert when the fifth determining module determines that the degree of movement is smaller than the movement threshold and the sixth determining module determines that the timer has expired. And/or, the inference module may further include a starting module configured to start the timer when the fifth determining module determines that the degree of movement is smaller than the movement threshold and the sixth determining module determines that the timer has not been started. And/or, the inference module may further include a resetting module configured to reset the timer when the fifth determining module determines that the degree of movement is greater than or equal to the movement threshold.

In particular, the timer may be a bad posture timer if the first determining module determines that the inference subject has the bad posture. Or the timer is a stillness timer if the first determining module determines that the inference subject has the good posture.

In still another example, the inference module further includes a seventh determining module configured to determine whether an activity timer has expired, and an eighth determining module configured to determine whether the inference subject has been sitting during an elapsed time of the activity timer based at least partially on the at least one second piece of information. The inference module further include a second rendering module configured to render an alert when the seventh determining module determines that the activity timer has expired and the eighth determining module determines that the inference subject has been sitting during the elapsed time of the activity timer based at least partially on the at least one second piece of information.

In still another example, the inference module further includes a receiving module configured to receive identity information of the inference subject, and a retrieving module configured to retrieve the distribution of information representing the class of poses of the inference subject using the identity information.

In still another example, the inference module further includes a ninth determining module configured to determine whether an activity timer has expired, and a third rendering module configured to render an alert when the ninth determining module determines that the activity timer has expired. Further, the inference module may include a tenth determining module configured to determine that the inference subject has been sitting during an elapsed time of the activity timer based at least partially on the at least one second piece of information, and the third rendering module is configured to render the alert under the condition that the tenth determining module determines that the inference subject has been sitting during the elapsed time of the activity timer.

The computing system according to the second aspect may include one or more processors, and one or more computer-readable storage medium storing instructions which, when executed by the one or more processors, cause the one or more processors to perform the method described above in the first aspect.

Consequently, in a third aspect, the disclosure is directed to a computer program performing the method presented in the first aspect when this program is executed by one or more processors.

This program can use any programming language and take the form of source code, object code or a code intermediate between source code and object code, such as a partially compiled form, or any other desirable form.

In a fourth aspect, the disclosure is directed to a computer-readable information medium containing instructions of the computer program as described above.

BRIEF DESCRIPTION OF THE DRAWINGS

Features, advantages, and technical and industrial significance of exemplary embodiments of the invention will be described below with reference to the accompanying drawings, in which like signs denote like elements, and wherein:

FIG. 1 schematically illustrates a work flow of a method 100 according to a first embodiment of the disclosure;
FIG. 2 schematically illustrates another work flow of the method 100 according to example implementations of the first embodiment;
FIG. 3 illustrates various joints of a user 300 according to various embodiments of the disclosure;
FIG. 4 schematically illustrates a computing system 400 according to various embodiments of the disclosure;
FIG. 5 schematically illustrates still another work flow of a process which is part of the method 100 according to a specific example of the first embodiment; and
FIG. 6 schematically illustrates a computing system 600 according to a second embodiment of the disclosure.

DETAILED DESCRIPTION OF EMBODIMENTS

We will now describe the invention in more detail.

A first embodiment of the present disclosure provides a method for posture determination. The method may be implemented by a computing system.

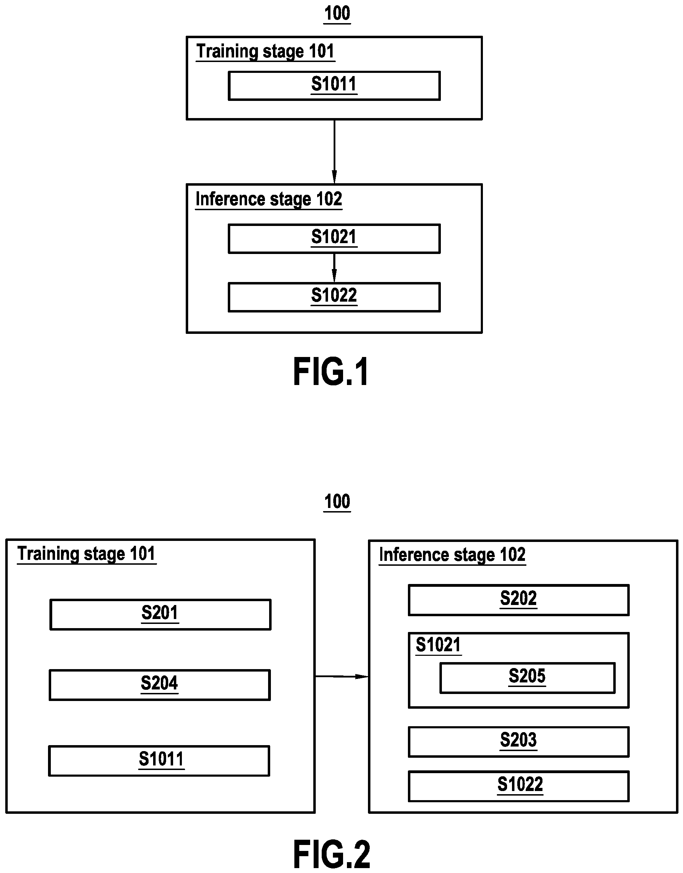

As illustrated by Fig. 1, the method 100 according to the first embodiment includes a training stage 101, which includes a generating step S 1011 of generating at least one distribution from first pieces of information representing a class of poses of one or more training subjects. The method 100 further includes an inference stage 102, which in turn includes a step S1021 of obtaining at least one second piece of information representing at least one pose of an inference subject, and a step S1022 of using a statistical anomaly detection technique to determine whether the inference subject has a good or bad posture based on the at least one second piece of information and the at least one distribution.

While Fig. 1 depicts stage 101 as preceding step S1021, it's worth noting that based on specific requirements, step S 1021 can alternatively be executed either before stage 101 or concurrently with it.

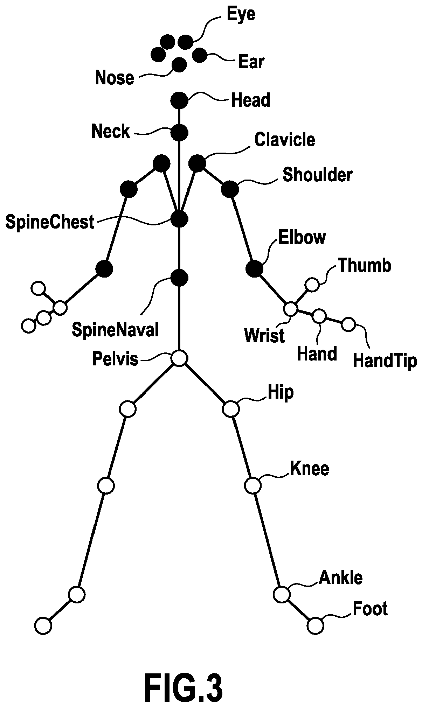

A specific example of the method 100 will be expounded upon below, based on a set of assumptions. It is presumed that N second pieces of information are obtained at the step S1021, with N being an integer greater than 1. Additionally, it is assumed that the first pieces of information represent a good sitting posture adopted by the inference subject - in this case, a human individual. In other words, the training subject and the inference subject are the same person, illustrated in Fig. 3 and hereinafter referred to as "the user 300". Moreover, it is presumed that N equals the size of a dimension of a 2D anomaly window and the size of a dimension of a 2D movement window. The number of second piece(s) of information by which a window moves or slides after processing each window is herein referred to as the "stride". In this example, the stride of the anomaly window and the movement window equals 1.

As used herein, the term "posture" refers to a spectrum of poses. These poses, characterized by minor variations that don't significantly alter the users state (like sitting, standing, or running), can be classified into categories such as "good" and "poor", based on certain standards, notably those of ergonomics.

A profile may be established by the computing system for the user 300, preferably at the step S201 in the training stage 101 that is illustrated in Fig. 2, if the computing system fails to locate the user 300's profile within any local or remote storage space and it can't retrieve the profile from any other sources. The profile may include identity information of the user 300 and the at least one distribution generated at the step S1011.

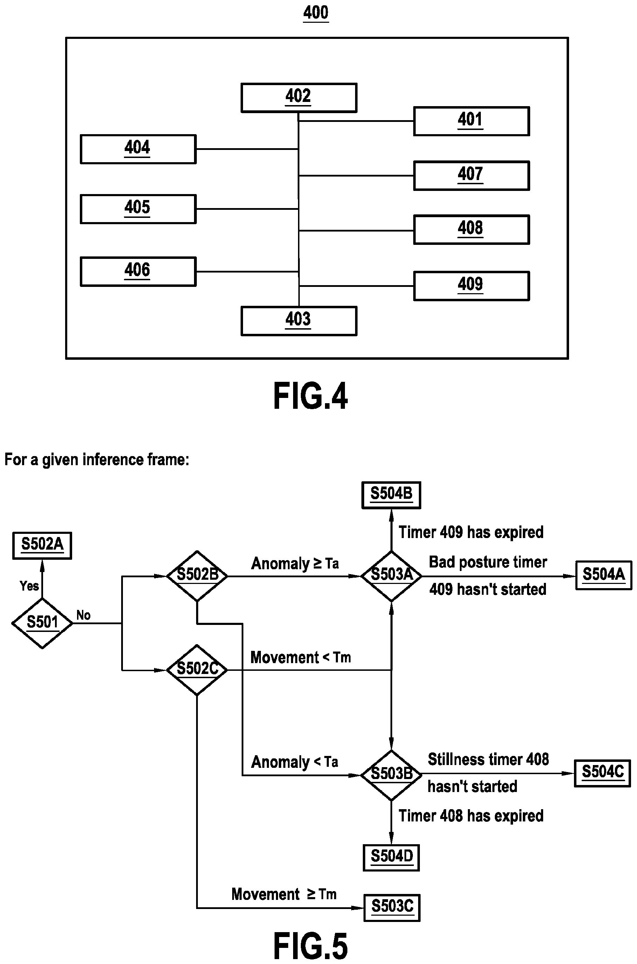

The system may be configured to access various users' profiles stored locally or remotely. Consequently, preferably prior to step S1021, the system may receive identity information of the user 300 at a step S202 and then use the identity information to retrieve the distribution of information representing the good sitting posture poses of the user 300 at a step S203. In particular, the system may be the system 400 as illustrated by Fig. 4, which incorporate one or more user interfaces 401 configured to receive identify information from users. These user interfaces 401 could include elements like a login form on a web portal, a biometric scanner for fingerprint or facial recognition, voice recognition software for vocal prompts, or even a quick response ("QR") code scanner for quick data transfer.

As illustrated by Fig. 4, the system 400 includes a camera 402 configured to capture poses of the user at the step S204 in the training stage 101 and at the step S205 in the inference stage 102. The camera 402 may be a color camera, such as one that is integrated into a laptop which the user 300 is utilizing, or a depth camera, such as the depth camera in any version of the Microsoft Kinect® motion sensing device. In this specific example, the camera 402 is the depth camera of a Microsoft Kinect® device, and livestreams video frames into the system 400.

In addition, during the training stage, the user 300 may be prompted to maintain a good sitting posture in order to obtain the first pieces of information. In contrast, during the inference stage, the user 300 is allowed to sit naturally. This progression enables the system to assist the user 300 in enhancing their sitting posture.

The first pieces of information include training images taken by the camera 402 at the step S204. The N second pieces of information include N inference images taken by the camera 402 at the step S205. As can be appreciated, the N inference images are associated with different time points: for example, they are taken at N different time points.

Various angles each associated with a joint of the user 300 are derived from each of the training images and the N inference images. In this example, the number of angles is 15, and the angles are associated with 15 joints in the upper body of the user 300. As is known in the art (e.g., in the realm of pose estimation), the term "joint" used herein does not necessarily correspond to its medical definition. Instead, it often refers to a critical body point in the skeleton that connects two body segments. Fig. 3 illustrates the example of the 32 joints tracked by Azure Kinect®, showing the joint locations and connection relative to the human body. The dots in darker color indicate the particular 15 joints utilized by the example implementations of the method, and the lines between the dots illustrate the body segments.

Accordingly, 15 distributions are generated at the step S 1011, each being a distribution of angles associated with a different joint of the user 300.

As illustrated by Fig. 4, the system 400 includes a pose-estimation model 403 configured to process the inference images and training images. In this specific example, the model 403 is the native pose estimation model of a Kinect® device. The system 400 derives the angles from the outputs of the pose-estimation model 403, which are normalized quaternions that represent the orientations of joints.

As illustrated by Fig. 4, the system 400 further includes a pose monitor 404 configured to track the poses of the user 300. The pose monitor 404 converts the 15 angles derived from each of the N inference images and training images to an anomaly score. In particular, the pose monitor 404 converts each of the 15 angles derived from a $j^{th}$ one of the N inference images to the anomaly score using the equation (1) below:

$$z_{ij} = (x_{ij} - u_i)/s_i \ (1),$$

where $z_{ij}$ represents the anomaly score of the $i^{th}$ angle $x_{ij}$, which is derived from the quaternion of the $i^{th}$ joint of the user 300, $u_i$ represents the mean of the distribution which includes the angles associated with the $i^{th}$ joint and is generated at the step S1011, and $s_i$ represents the standard deviation of the distribution.

The pose monitor 404 further converts the 15 anomaly scores derived from each of the N inference images and training images to a single average anomaly score. In addition, the pose monitor 404 may insert the 15 anomaly score of each of the N inference images to a corresponding column of the anomaly window of a size 15 × N, then convert each row of the anomaly window to a mean score. For instance, the mean score of an $i^{th}$ row of the anomaly window, which corresponds to the $i^{th}$ angles derived from the quaternions of the $i^{th}$ joint of the user 300, is equivalent to $z_i$ below:

$$z_i = \frac{1}{N}\sum_{j=1}^{N} x_{ij} \ (2).$$

The pose monitor 404 then calculates a weighted average of the 15 mean scores as an average anomaly score.

Consequently, at the step S 1022, the pose monitor 404 determines that the user 300 has the good sitting posture if the average anomaly score is smaller than an average anomaly threshold, or determines that the inference subject has the bad posture if the average anomaly score is equal to or greater than the average anomaly threshold.

The average anomaly threshold is greater than a first maximum anomaly score derived from the 15 distributions. In this specific example, since 3 standard deviations encapsulate 99.7% of the total angle distribution from the training, 3.5 is selected as the average anomaly threshold to capture the total distribution while adding a slight amount of bias to reduce false positives.

The weight of each of the 15 mean scores depends on the degree that the angle associated with the corresponding weighs on the bad posture. For instance, certain body parts like the neck and back may require more immediate correction compared to the arms. In this specific example, higher weights are assigned to those angles whose corresponding joints contribute more to the bad posture than other joints.

In addition, when determining that the user 300 has the bad posture, the pose monitor 404 compares the mean score of each row of the anomaly window to an anomaly threshold associated with the corresponding joint. The corresponding anomaly threshold is greater than a second maximum anomaly score derived from the corresponding distribution of the training image. Accordingly, the pose monitor 404 determines the particular body parts of the user 300 which contribute to the bad posture.

As illustrated by Fig. 4, the system 400 further includes a movement monitor 405 configured to track the movement of the user 300. In this specific example, the N inference images are taken at N different time points, and the movement monitor 405 determines a degree of movement of the user 300 by determining a degree of difference between the pose represented by the inference image taken at the final time point of the N different time points and an average of the poses represented by the N inference images.

More specifically, the movement monitor 405 inserts the 15 angles derived from each of the N inference images to a corresponding column of the movement window of a size 15 × N, and determines a weighted average of variances, $o^2$, as the above-described degree of difference, using the equation (3) below:

$$o^2 = \sum_{i=1}^{K} w_i \left(x_{if} - v_i\right)^2 / N \quad (3),$$

where $x_{if}$ represents the $i^{th}$ angle associated with the $i^{th}$ joint of the user 300 that is derived from the inference image taken at the final time point, $v_i$ represents the average of all the $i^{th}$ angles derived from the N inference images across the different time points, $w_i$ represents the weight assigned to the $i^{th}$ joint, and K represents the number of angles, which is 15.

As illustrated by Fig. 4, the system 400 further includes an activity monitor 406 configured to track the activity of the user 300, an activity timer 407, a stillness timer 408 and a bad posture timer 409. The workflow of the three monitors 404-406 is delineated below.

As illustrated by Fig. 5, for every subsequent inference frame obtained after the first (N-1) inference frames (or inference images), the activity monitor 406 evaluates, at the step S501, whether the activity timer 407 has expired. If it has, the activity monitor 406 generates an alert at the step S502A to prompt the user 300 to move around. If there is still time left on the timer 407, both the pose monitor 404 and the movement monitor 405 continue to execute their respective operations.

Moreover, the pose monitor 404 compares the average anomaly score of the past N frames with the average anomaly threshold at the step S502B, and the movement monitor 405 compares the degree of movement for the past N frames with a movement threshold at the step S502C.

If the average anomaly score is higher than or equal to the average anomaly threshold Ta, and the degree of movement is lower than the movement threshold Tm, then the system 400 checks the state of the bad posture timer 409 at the step S503A. The system 400 starts the bad posture timer 409 at the step S504A if this timer 409 has not been started, or alerts the user 300 to the bad posture at the step S504B if the timer 409 has expired. The alert includes information identifying the particular body parts which contribute to the bad posture.

If the average anomaly score is lower than the average anomaly threshold Ta, and the degree of movement is lower than the movement threshold Tm, then the system 400 checks the state of the stillness timer 408 at the step S503B. The system 400 starts the stillness timer 408 at the step S504C if the timer 408 has not been started, or alerts the user 300 that they have been sitting still for too long and recommends that they move around at the step S504D if the timer 408 has expired.

If the degree of movement is higher than or equal to the movement threshold Tm, the system 400 resets the timers 408-409 at the step S503C.

The system 400 then continues to process the next inference frame.

As can be appreciated, the method 100 can be implemented for different inference subjects concurrently.

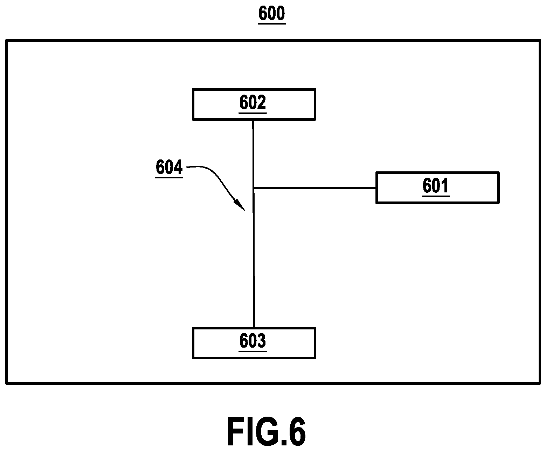

A second embodiment of the present disclosure provides a computing system for implementing the method 100 according to the first embodiment. As illustrated by Fig. 6, the system 600 includes one or more processors represented by the processor 601, and one or more computer-readable storage media represented by the storage medium 602. The one or more storage media store instructions which, when executed by the one or more processors, cause the one or more processors to perform the method 100 according to the first embodiment. As can be appreciated, the system 600 may be the same as the system 400.

As an example, the interference subject may be a driver or a passenger in a vehicle such as an automobile. The method 100 may continuously monitor the posture of the inference subject in real-time, providing alerts based on the monitored data. Accordingly, the computing system 400 or 600 may be integrated into or externally connected to the vehicle.

Examples of the processor 601 include a central processing unit (hereinafter "CPU"), a vision processing unit (hereinafter "VPU"), a graphics processing unit (hereinafter "GPU"), a tensor processing unit (hereinafter "TPU"), a neural processing unit (hereinafter "NPU"), a neural processing engine, a core of a CPU, VPU, GPU, TPU, NPU or another processing device, an application processor, a display controller, an application specific integrated circuit (hereinafter "ASIC"), a field programmable gate array (hereinafter "FPGA"), a coprocessor, or any other hardware configured to function as a processing unit.

The storage medium 602 can be any available medium that can be accessed by the computing system 600 in the form of volatile or non-volatile memory. Examples of the storage medium 602 includes a random access memory (hereinafter "RAM"), a dynamic random access memory (hereinafter "DRAM"), a static random access memory (hereinafter "SRAM"), any other form of volatile memory known in the art, a magnetic hard disk, an optical disk, a floppy disk, a flash memory, an electrically programmable memory (hereinafter "EPROM"), an electrically erasable and programmable memory (hereinafter "EEPROM"), any other form of non-volatile memory known in the art, a data server, etc.

As illustrated by Fig. 6, the computing system 600 may further include a notification system 603 that is configured to display alerts. This might involve showing a popup window or notification on a screen, lighting up a light emitting diode (hereinafter "LED"), vibrating a device, playing a sound, writing a message to a log file, or sending an email or text message to an administrator. The notification system could be in software and/or hardware.

As shown by FIG. 6, the processor 601, the storage medium 602 and the notification system 603 may be directly or indirectly coupled to each other physically, communicatively, or operationally via a communication channel 604. The communication channel 604 may include one or more buses (such as an address bus, data bus or combination thereof), a network connection, an inter-process communication data structure, or any other means for communicating data.

The computing system 600 may include a high-performance computer, a server, a user device (e.g., a laptop computer, a home desktop computer, a mobile device such as a tablet, a smart phone, a wearable device, etc.), an embedded device (e.g., a device embedded within a vehicle, a camera, an image sensor, a household appliance, etc.), a platform having one or more corresponding application programming interfaces (hereinafter "APIs"), a cloud infrastructure, or any other computing device suitable for perform one or more steps of the method 100 according to the first embodiment.

A third embodiment of the present disclosure provides a computer-readable information medium containing instructions which, when executed by one or more processors, enable the one or more processors to perform the method 100 according to the first embodiment.

The information medium may be the same as the storage medium 602 described above. Alternatively, the information medium can be an integrated circuit in which the instructions are incorporated, the circuit being adapted to execute the method 100 or to be used in its execution.

**Claims**

1. A computer-implemented method (100) for posture determination, comprising:

   a training stage (101) of generating (S 1011) at least one distribution from first pieces of information representing a class of poses of one or more training subjects; and
   an inference stage (102) comprising steps of:

   obtaining (S1021) at least one second piece of information representing at least one pose of an inference subject; and
   determining (S 1022) whether the inference subject has a good or bad posture using a statistical anomaly detection technique based on the at least one second piece of information and the at least one distribution.

2. The method according to claim 1, wherein the first pieces of information represent the class of poses of one training subject, and, the training subject and the inference subject are the same subject; or,

wherein the class of poses represents one or more good postures adopted by the one or more training subjects; or,

a quantity of the at least one second piece of information equals a size of a dimension of an anomaly window.

3. The method according to claim 1 or 2, wherein the determining step (S 1022) comprises:

converting the at least one second piece of information to at least one anomaly score based on the at least one distribution; and

determining whether the inference subject has the good posture or the bad posture based on the at least one anomaly score.

4. The method according to claim 3, wherein the converting step comprises:

deriving at least one feature each associated with a key body part, also referred to as a joint, of the inference subject, from the at least one second piece of information; and

converting the at least one feature to the at least one anomaly score based on the at least one distribution.

5. The method according to claim 4, wherein converting the at least one feature to the at least one anomaly score comprises:

converting one of the at least one feature that is associated with an $i^{th}$ joint of the inference subject into a joint-specific anomaly score based on a relationship between a standard deviation of a distribution comprised in the at least one distribution and a difference between the feature and a mean of the distribution, wherein the distribution comprises features associated with the $i^{th}$ joint of the one or more training subjects.

6. The method according to claim 4 or 5, wherein

the deriving step comprises: analyzing the at least one second piece of information using a pose estimation technique to produce at least one result, and deriving the at least one feature from the at least one result; or

the at least one feature comprises at least one angle; or,

converting the at least one feature to the at least one anomaly score comprises:

converting the features to an average anomaly score equaling an average of base values that is incorporated with weights, wherein each of the base values is determined by one of the features and one of the distributions, and the distribution and the feature are associated with a same joint.

7. The method according to claim 6, wherein a number of the obtained at least one second piece of information is N, K features are derived from each of the N second pieces of information, N and K are integers greater than 1, and converting the features to the average anomaly score comprises:

converting an $i^{th}$ feature that is associated with an $i^{th}$ joint of K joints of the inference subject and is derived from one of the N second pieces of information to a joint-specific anomaly score corresponding to the $i^{th}$ joint;

calculating K mean scores each equaling an average of N joint-specific anomaly scores corresponding to one of the K joints of the inference subject; and

calculating the average anomaly score, wherein each of the base values is one of the K mean scores.

8. The method according to claim 6 or 7, wherein the weight of each of the base values depends on a degree that the corresponding feature weighs on the bad posture.

9. The method according to any one of the claims 3 to 8, wherein the determining step based on the at least one anomaly score comprises:

determining whether the inference subject has the good posture or the bad posture based on at least one relationship between the at least one anomaly score and the at least one anomaly threshold, wherein

the at least one anomaly score is an average anomaly score, the at least one anomaly threshold is an average anomaly threshold, and the average anomaly threshold is greater than a first maximum anomaly score derived from the at least one distribution; or,

the at least one anomaly score comprises joint-specific anomaly scores each corresponding to a different joint of the inference subject, the at least one anomaly threshold comprises joint-specific anomaly thresholds each corresponding to a different joint of the inference subject, and each of the joint-specific anomaly thresholds is greater than a second maximum anomaly score derived from one of the at least one distribution, wherein the joint-specific anomaly threshold and the distribution are associated with a same joint.

10. The method according to any one of the claims 1 to 9, wherein the inference stage (102) further comprises:

determining a degree of movement of the inference subject based on P second pieces of information that represent P poses of the inference subject, P being an integer greater than 1; and
comparing the degree of movement with a movement threshold.

11. The method according to claim 10, wherein the P second pieces of information are associated with P different time points, and determining the degree of movement comprises:
determining a degree of difference between a pose represented by one of the P second pieces of information that is associated with a final time point of the P different time points, and an average of the P poses represented by the P second pieces of information.

12. The method according to claim 11, wherein the degree of difference is represented by an average of variances that is incorporated with weights, each of the variances measures a degree of difference between a feature that is associated with one of K different joints of the inference subject and is derived from the second piece of information associated with the final time point and an average feature that is associated with the joint of the inference subject and is derived from the P second pieces of information, and K is an integer greater than 1.

13. The method according to any one of the claims 10 to 12, wherein the inference stage (102) further comprises:
rendering (S504B, S504D) an alert when determining that the degree of movement is smaller than the movement threshold and a timer (409, 408) has expired, or, starting (S504A, S504C) the timer (409, 408) when determining that the degree of movement is smaller than the movement threshold and the timer has not been started, or, resetting (503C) the timer (409, 408) when determining that the degree of movement is greater than or equal to the movement threshold.

14. The method according to claim 13, wherein the timer is a bad posture timer (409) if a result of the step of determining (S1022) whether the inference subject has the good posture or the bad posture indicates that the inference subject has the bad posture, or the timer is a stillness timer (408) if the result indicates that the inference subject has the good posture.

15. The method according to any one of the claims 1 to 14, wherein the inference stage (102) further comprises:
rendering (S502A) an alert when determining that an activity timer (407) has expired.

16. A computing system (600), comprising:

one or more processors (601); and
one or more computer-readable storage media (602) storing instructions which, when executed by the one or more processors (601), cause the one or more processors (601) to perform the method according to any one of the claims 1 to 15.

**Amended claims in accordance with Rule 137(2) EPC.**

1. A computer-implemented method (100) for posture determination, comprising:

a training stage (101) of generating (S1011) at least one distribution from first pieces of information representing a class of poses of one or more training subjects, the at least one distribution each being a distribution of angles associated with a joint of the one or more training subjects, the first pieces of information each comprising an image obtained by using a camera, the class of poses representing a good posture adopted by the one or more training subjects; and
an inference stage (102) comprising steps of:

obtaining (S1021) at least one second piece of information representing at least one pose of an inference subject, the at least one second piece of information each comprising an image obtained by using a camera; and

determining (S1022) whether the inference subject has the good posture or a bad posture using a statistical anomaly detection technique based on the at least one second piece of information and the at least one distribution,
wherein the determining step (S1022) comprises:

converting the at least one second piece of information to at least one anomaly score based on the at least one distribution; and determining whether the inference subject has the good posture or the bad posture based on the at least one anomaly score,
wherein the converting step comprises:

deriving at least one angle each associated with a joint of the inference subject from the at least one second piece of information; and converting the at least one angle to the at least one anomaly score based on the at least one distribution,
wherein converting the at least one angle to the at least one anomaly score comprises:

converting the angles to an average anomaly score equaling a weighted average of base values, wherein each of the base values is determined by one of the angles and one of the distributions, and the distribution and the angle are associated with a same joint,
wherein the number of the obtained at least one second piece of information is N, K angles are derived from each of the N second pieces of information, N and K are integers greater than 1, and converting the angles to the average anomaly score comprises:

converting an $i^{th}$ angle that is associated with an $i^{th}$ joint of K joints of the inference subject and is derived from one of the N second pieces of information to a joint-specific anomaly score corresponding to the $i^{th}$ joint; calculating K mean scores each equaling an average of N joint-specific anomaly scores corresponding to one of the K joints of the inference subject; and calculating the average anomaly score,
wherein each of the base values is one of the K mean scores, the $K \times N$ joint-specific anomaly scores is inserted into an anomaly window that is a two-dimensional array of size $K \times N$ or $N \times K$, and each element of the array represents one of the joint-specific anomaly scores.

2. The method according to claim 1, wherein the first pieces of information represent the class of poses of one training subject, and, the training subject and the inference subject are the same subject; or,
the quantity of the at least one second piece of information equals size of a dimension of the anomaly window, wherein the anomaly window is a sliding window shifting one or more second pieces of information at a time, and the number of the one or more second pieces of information is smaller than or equals to the size of the dimension of the sliding window.

3. The method according to claim 1 or 2, wherein converting the $i^{th}$ angle that is associated with the $i^{th}$ joint of the inference subject and is derived from one of the N second pieces of information to the joint-specific anomaly score corresponding to the $i^{th}$ joint comprises:
converting the angle into a joint-specific anomaly score by dividing a difference between the angle and a mean of a distribution comprised in the at least one distribution by a standard deviation of the distribution, wherein the distribution comprises angles associated with the $i^{th}$ joint of the one or more training subjects.

4. The method according to any one of the claims 1 to 3, wherein the deriving step comprises:
analyzing the at least one second piece of information using a pose estimation technique to produce at least one result, and deriving the at least one angle from the at least one result.

5. The method according to any one of the claims 1 to 4, wherein the determining step based on the at least one anomaly score comprises:
determining whether the inference subject has the good posture or the bad posture based on at least one relationship between the average anomaly score and an average anomaly threshold, wherein the average anomaly threshold is greater than a maximum standard score required to encompass majority of data points across the at least one distribution.

6. The method according to any one of the claims 1 to 5, wherein the inference stage (102) further comprises:

determining a degree of movement of the inference subject based on P second pieces of information that represent P poses of the inference subject, P being an integer greater than 1; and

comparing the degree of movement with a movement threshold.

7. The method according to claim 6, wherein the P second pieces of information are associated with P different time points, and determining the degree of movement comprises:
determining a degree of difference between a pose represented by one of the P second pieces of information that is associated with a final time point of the P different time points, and an average of the P poses represented by the P second pieces of information.

8. The method according to claim 7, wherein the degree of difference is represented by a weighted average of variances, each of the variances measures a degree of difference between an angle that is associated with one of the K different joints of the inference subject and is derived from the second piece of information associated with the final time point and an average angle that is associated with the joint of the inference subject and is derived from the P second pieces of information.

9. The method according to any one of the claims 6 to 8, wherein the inference stage (102) further comprises:
rendering (S504B, S504D) an alert when determining that the degree of movement is smaller than the movement threshold and a timer (409, 408) has expired, or, starting (S504A, S504C) the timer (409, 408) when determining that the degree of movement is smaller than the movement threshold and the timer has not been started, or, resetting (503C) the timer (409, 408) when determining that the degree of movement is greater than or equal to the movement threshold.

10. The method according to claim 9, wherein the timer is a bad posture timer (409) if a result of the step of determining (S1022) whether the inference subject has the good posture or the bad posture indicates that the inference subject has the bad posture, or the timer is a stillness timer (408) if the result indicates that the inference subject has the good posture.

11. The method according to any one of the claims 1 to 10, wherein the inference stage (102) further comprises:
rendering (S502A) an alert when determining that an activity timer (407) has expired.

12. A computing system (600), comprising:

one or more processors (601); and
one or more computer-readable storage media (602) storing instructions which, when executed by the one or more processors (601), cause the one or more processors (601) to perform the method according to any one of the claims 1 to 11.

**100**

```
Training stage 101
    ┌─────────────────────┐
    │       S1011         │
    └─────────────────────┘

            │
            ▼

Inference stage 102
    ┌─────────────────────┐
    │       S1021         │
    └─────────────────────┘
            │
            ▼
    ┌─────────────────────┐
    │       S1022         │
    └─────────────────────┘
```

# FIG.1

**100**

```
Training stage 101              Inference stage 102
 ┌──────────────┐                ┌──────────────┐
 │    S201      │                │    S202      │
 └──────────────┘                └──────────────┘

 ┌──────────────┐                S1021
 │    S204      │         ──→     ┌──────────────┐
 └──────────────┘                 │    S205      │
                                  └──────────────┘
 ┌──────────────┐
 │    S1011     │                ┌──────────────┐
 └──────────────┘                │    S203      │
                                 └──────────────┘
                                 ┌──────────────┐
                                 │    S1022     │
                                 └──────────────┘
```

# FIG.2

FIG.3

<u>400</u>

# FIG.4

For a given inference frame:

# FIG.5

600

602

604

601

603

FIG.6

Europäisches
Patentamt
European
Patent Office
Office européen
des brevets

# EUROPEAN SEARCH REPORT

**Application Number**

EP 23 18 2186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/129666 A1 (CHERIAN ANOOP [US] ET AL) 28 April 2022 (2022-04-28) | 1-4,6,16 | INV.<br>G06V10/62 |
| Y | * paragraphs [0007] – [0009]; figure 4 *<br>* paragraph [0050] *<br>* paragraphs [0094] – [0097]; figure 6 * | 10-15 | G06V40/10<br>G06V40/20 |
| X | VOINEA GHEORGHE-DANIEL ET AL: "Vision-Based System for Driver Posture Tracking to Prevent Musculoskeletal Disorders", 2020 INTERNATIONAL CONFERENCE ON E-HEALTH AND BIOENGINEERING (EHB), IEEE, 29 October 2020 (2020-10-29), pages 1-4, XP033869400, DOI: 10.1109/EHB50910.2020.9280263 [retrieved on 2020-12-02] | 1-9,16 | |
| Y | * the whole document * | 10-15 | |
| A | US 2020/205697 A1 (ZHENG JIANNAN [CA] ET AL) 2 July 2020 (2020-07-02) * abstract * | 1-16 | |
| | | | **TECHNICAL FIELDS SEARCHED (IPC)** |
| Y | RODRIGUES PATRICK B ET AL: "Ergonomic assessment of office worker postures using 3D automated joint angle assessment", ADVANCED ENGINEERING INFORMATICS, ELSEVIER, AMSTERDAM, NL, vol. 52, 30 March 2022 (2022-03-30), XP087084179, ISSN: 1474-0346, DOI: 10.1016/J.AEI.2022.101596 [retrieved on 2022-03-30] * the whole document * | 10-15 | G06V |

-/--

The present search report has been drawn up for all claims

| Place of search | Date of completion of the search | Examiner |
|---|---|---|
| The Hague | 30 November 2023 | Neubüser, Bernhard |

EPO FORM 1503 03.82 (P04C01)

page 1 of 2

Europäisches
Patentamt

European
Patent Office

Office européen
des brevets

# EUROPEAN SEARCH REPORT

Application Number

EP 23 18 2186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Ergonomic Risk Analysis – viso.ai", , 4 May 2023 (2023-05-04), pages 1-4, XP093106519, Retrieved from the Internet: URL:https://viso.ai/application/ergonomics -analysis/ [retrieved on 2023-11-28] * the whole document * ----- | 1-16 | |
| A | CN 111 597 974 A (HRG INT INST RES & INNOVATION) 28 August 2020 (2020-08-28) * the whole document * ----- | 1-16 | |

TECHNICAL FIELDS
SEARCHED    (IPC)

The present search report has been drawn up for all claims

| Place of search | Date of completion of the search | Examiner |
|---|---|---|
| The Hague | 30 November 2023 | Neubüser, Bernhard |

EPO FORM 1503 03.82 (P04C01)

page 2 of 2

21

## ANNEX TO THE EUROPEAN SEARCH REPORT
## ON EUROPEAN PATENT APPLICATION NO.

EP 23 18 2186

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2023

| Patent document cited in search report | | | Publication date | Patent family member(s) | | | Publication date |
|---|---|---|---|---|---|---|---|
| US 2022129666 | A1 | | 28-04-2022 | CN | 116348924 | A | 27-06-2023 |
| | | | | EP | 4026051 | A1 | 13-07-2022 |
| | | | | JP | 2023542058 | A | 04-10-2023 |
| | | | | US | 2022129666 | A1 | 28-04-2022 |
| | | | | WO | 2022091494 | A1 | 05-05-2022 |
| US 2020205697 | A1 | | 02-07-2020 | CN | 111383421 | A | 07-07-2020 |
| | | | | CN | 112784662 | A | 11-05-2021 |
| | | | | US | 2020205697 | A1 | 02-07-2020 |
| | | | | US | 2020211154 | A1 | 02-07-2020 |
| | | | | US | 2021378554 | A1 | 09-12-2021 |
| | | | | US | 2022079472 | A1 | 17-03-2022 |
| CN 111597974 | A | | 28-08-2020 | NONE | | | |

EPO FORM P0459

For more details about this annex : see Official Journal of the European Patent Office, No. 12/82